# EUROPÄISCHE PATENTSCHRIFT

(11) **EP 1 257 823 B1**
(45) Veröffentlichungstag und Bekanntmachung des Hinweises auf die Patenterteilung: **04.02.2004**
(21) Anmeldenummer: 01919280.6
(22) Anmeldetag: 14.02.2001
(51) Int. Cl.: G01N 33/569

(54) **VERFAHREN ZUM NACHWEIS VON HELICOBACTER PYLORI UND HEILMANII**
METHOD FOR DETECTING HELICOBACTER PYLORI AND HEILMANII IN FECAL AND SALIVARY SPECIMEN AND BIOPSY MATERIAL
PROCEDE DE DETECTION DE HELICOBACTER PYLORI ET HEILMANII

(30) Priorität: 14.02.2000 DE 10006432
(43) Veröffentlichungstag der Anmeldung: 20.11.2002
(73) Patentinhaber: Immundiagnostik AG, 64625 Bensheim (DE); Ganzimmun Ag, 55128 Mainz (DE)
(72) Erfinder: ARMBRUSTER, Franz, Paul, Immundiagnostik AG, 64625 Bensheim (DE); CREVAR, Katarina, 64832 Babenhausen (DE); RUPPERT, Jana, 64682 Lautertal (DE)
(74) Vertreter: Benedum, Ulrich Max, Dr.
(86) Internationale Anmeldenummer: PCT/EP2001/001639
(87) Internationale Veröffentlichungsnummer: WO 2001/063285

(56) Entgegenhaltungen:
- EP-A- 0 329 570
- WO-A-93/22682
- WO-A-98/27432
- WO-A-99/41611

## Beschreibung

Die Erfindung betrifft ein Verfahren zum Nachweis von Helicobacter-Antigenen in Stuhl- und Speichelproben oder Biopsiematerialien.

Die menschliche Magenschleimhaut wird vielfach von Bakterien der Gattungen *Helicobacter pylori und heilmanii* oder *Campylobacter* besiedelt. Die Infektion mit diesen Bakterien erfolgt wahrscheinlich von Mensch zu Mensch, aber auch Trinkwasser und Nahrungsmittel werden als Infektionsquellen nicht ausgeschlossen. Der Stamm *Helicobacter heilmanii* wird von Haustieren wie Katzen, Hunden, Kaninchen, aber auch von Nutztieren wie Kühen übertragen. Daher sind Personen in der Landwirtschaft und Tierpflege besonders von Infektionen betroffen. In Deutschland sind etwa 10% der Schulkinder, 30% der Dreißigjährigen und etwa 75% der Senioren mit *H.pylori* infiziert. Weltweit tragen ca. 50% der Menschen diese Infektion. 80% der Gastriden, 95% der Ulcus duodeni- und 70% der Ulcus ventriculi-Fälle werden von *H. pylori* verursacht. Zudem gilt die chronische *H.pylori*-Gastritis (Typ-B-Gastritis) als Präkanzerose für das Magen-Adenokarzinom und das gastrische Lymphom. *H. pylori* ist gemäß WHO ein Kanzerogen der höchsten Krebsrisikoklasse. Nur ein kleiner Teil der *H.pylori*-infizierten Personen entwickelt Symptome. Viele leben trotz einer Gastritis ohne nennenswerte Beschwerden oder sie schreiben die eher unspezifischen Symptome anderen Ursachen zu. Eine akute *H.pylori*-Gastritis äußert sich durch unbestimmte Schmerzen im Ober- und Mittelbauch, Druck- und Völlegefühl, saures Aufstoßen, Sodbrennen sowie durch Übelkeit und Brechreiz. Bei nahezu allen Patienten mit einer Typ-B-Gastritis kann eine *H. pylori-* Infektion nachgewiesen werden. Trotz der zumeist massiven Immunreaktion wurde bei ihnen die Infektion chronisch. Ob sich ein Ulkus entwickelt, hängt ab vom Immunsystem des Patienten und von der Aggressivität der Bakterien bzw. der Art des Bakterienstammes. Spontanheilungen sind selten. In der Regel bleibt eine H.pylori-Infektion wenn sie nicht therapiert wird, das ganze Leben bestehen.

Eine *H. pylori*-Infektion kann diagnostiziert werden durch Anzucht des Erregers aus einer Antrum- oder Corpus-Biopsie oder durch histologische Untersuchung des Gewebes. Weitere Diagnoseverfahren sind der CLO-Test (Test auf campylobacter like organism) bzw. der Urease-Harnstofftest, der ¹³C-Isotopen-Atemtest, der Nachweis von Antikörpern gegen *H.pylori* im Serum, der PCR-Nachweis von Helicobacter-DNA in einer Magensaft- oder Stuhlprobe und der Nachweis von *H.pylori*-Antigenen in einer Stuhlprobe.

US 5 716 791 (Larka et al.) und EP 0 806 667 (Meridian Diagnostics Inc.) beschreiben einen Immunoassay für *H.pylori*-Antigene in Stuhl. Der Assay beruht auf zwei affinitätsgereinigten polyklonalen Antikörpern gegen H. pylori-Antigen. Ferner gibt es von Connex GmbH, Martinsried, DE, einen Instanttest, der auf einer Lateralfluss-Chromatographie goldmarkierter monoklonaler Antikörper gegen *H. pylori*-Stuhlantigene basiert. Diese sogenannten HpSA-Tests (**H**elicobacter **p**ylori **S**tool **A**ntigen) haben zwar in verschiedenen klinischen Studien eine gute Übereinstimmung mit anderweitigen diagnostizierten Fällen ergeben, ein hoher Prozentsatz der Untersuchungen führt aber zu keiner klaren Aussage. Der HpSA-Test ist zudem für die Kontrolle einer Eradikationstherapie ungeeignet, da er nur bei abundanten Mengen *H. pylori*-Antigen im Stuhl funktioniert. Die weiteren diagnostischen Verfahren sind zum Teil sehr aufwendig, für den Patienten belastend oder zu teuer für Routineuntersuchungen. Nachteilig an den serologischen Verfahren ist, dass sie keine Therapiekontrolle erlauben, da die Antikörpertiter noch Monate nach einer Eradikation der Bakterien hoch bleiben. Eine Kontrolle der Therapie ist aber essentiell, da gegen die zur Behandlung üblicherweise eingesetzten Antibiotika wie Clarithromycin, Metronidazol, Amoxicillin, Omeprazol oder Protonenpumpenhemmer Resistenzen vorliegen können. Die Therapie der Infektionen durch Antibiotika und naturheilkundlichen Alternativen verlangt somit ein einfaches, zuverlässiges und empfindliches Testverfahren auf *H.pylori*.

Diese Aufgabe wird gelöst durch einen Immunoassay gemäß Anspruch 1. Bevorzugte Ausführungsformen der Erfindung sind in den Unteransprüchen beschrieben.

Das erfindungsgemäße Verfahren zum Nachweis von Helicobacter-Erregerantigenen in einer Probe ist gekennzeichnet durch das Aufnehmen und Suspendieren einer auf den Erreger zu untersuchenden menschlichen Probe in einem Probenpuffer; das Zusammenbringen des Probenpuffers mit den Erregerantigen mit einer festen Phase, an der mindestens zwei verschiedene Primärantikörper gebunden sind, von denen einer Antigene des Erregers und der andere menschliches Immunglobulin zu binden vermag; Waschen der festen Phase von unspezifisch gebundenen Antigenen; Zusammenbringen der festen Phase mit einem Sekundärantikörper, der spezifisch Antigene des Erregers erkennt und Bestimmen der Menge an gebundenem Sekundärantikörper.

Die Menge des an die feste Phase gebundenen Sekundärantikörpers kann bestimmt werden beispielsweise durch eine Markierung des Antikörpers. Die Markierung kann eine Radioaktivität sein, eine lumineszierende oder fluoreszierende Gruppe, eine Gruppe wie Biotin, die von einem weiteren Molekül wie Streptavidin gebunden werden kann, oder ein Enzym wie Peroxidase, das eine Nachweisreaktion katalysiert. Die Menge an gebundenem Sekundärantikörper kann auch durch einen weiteren Antikörper bestimmt werden, der den Typ des Sekundärantikörpers spezifisch erkennt und eine der vorgenannten Markierungen trägt.

Der an der festen Phase gebundene zweite Primärantikörper gegen menschliche Immunglobuline ist bevorzugt ein Antikörper, welcher Human-IgA und besonders bevorzugt sekretorisches Human-IgA bindet, das in den Speichel und im Dickdarm sekretiert wird. Die Bezeichnung sekretorisches Human-IgA umfasst hier Human-IgA und ggf. auch die sogenannte sekretorische Komponente (MW: 70 kDa), die von den Epithelzellen für den Transport und zum Schutz des IgA vor proteolytischen Enzymen produziert wird. Besonders bevorzugt sind Antikörper gegen IgA2, da Helicobacter und auch andere Mikroorganismen sIgA1-spaltende Proteasen erzeugen. Ganz besonders bevorzugt ist ein Gemisch zweier Primärantikörper, welche IgA1 und IgA2 spezifisch binden.

In einer bevorzugten Ausführungsform der Erfindung ist der an der festen Phase gebundene erst Primärantikörper ein polyklonaler Kaninchen-Anti-*H.pylori*-Antikörper. Der zweite an der festen Phase gebundene Primärantikörper ist ein auf Kreuzreaktivitäten getesteter polyklonaler Ziege-Anti-Human-sIgA-Antikörper. Der Sekundärantikörper ist ein Biotin-konjugierter Kaninchen-Anti-*H.pylori*-Antikörper, so dass die Menge an gebundenem Sekundärantikörper über die Bindung von Peroxidase-konjugiertem Streptavidin und eine Farbreaktion mit Tetramethylbenzidin bestimmt werden kann.

In einer zweiten bevorzugten Ausführungsform ist der erste an der festen Phase gebundene Primärantikörper ein Kaninchen-anti-*H.pylori*-Antikörper, der zweite Primärantikörper ein Kaninchen-anti-Human-sIgA-Antikörper und der Sekundärantikörper ein polyklonaler Meerrettichperoxidase-konjugierter Ziege-Anti-*H.pylori*-Antikörper. Es können aber auch andere Immunglobuline verwendet werden aus Pferd, Rind, Schwein, Schaf, Ziege, Kaninchen, Meerschweinchen, Ratte, Maus oder einem anderen Tier. Es ist besonders darauf zu achten, dass der zweite Primärantikörper für menschliches Immunglobulin nicht den Sekundärantikörper gegen das Erregerantigen in der Speichel- oder Stuhlprobe bindet. Die Antikörper können im Prinzip monoklonal sein. Insbesondere kann der zweite Primärantikörper ein monoklonaler Antikörper für Human-IgA2 sein. Es empfiehlt sich, mehrere monoklonale Antikörper zu verwenden, die verschiedene Epitope des Erregers bzw. der menschlichen Immunglobuline erkennen. Bei monoklonalen Primärantikörpern empfiehlt es sich, eine Mischung aus mehreren monoklonalen Antikörpern zu verwenden, um die Spezifität des Assays zu verbreitern.

Die Sekundärantikörper sind bevorzugt mit ein oder mehreren Markern konjugiert wie Biotin, Fluroescein, Rhutenium, Europium, Goldpartikel, alkalischer Phosphatase, Galactosidase oder Meerrettichperoxidase. Es können aber auch andere geeignete Marker- bzw. Nachweissysteme verwendet werden.

### Testprinzip am Beispiel eines ELISA

Der erfindungsgemäße ELISA (enzyme-linked immunosorbens assay) dient der qualitativen und quantitativen Bestimmung von *H.pylori*-Antigen in Stuhl- und Speichelproben und Biospiematerial. Das *H.pylori*-Antigen wird in einem ersten Schritt aus der Probe freigesetzt und durch einen bevorzugt polyklonalen Anti-*H.pylori*-Antikörper gebunden, welcher in üblicher Weise an eine Mikrotiterplatte oder eine andere feste Phase gebunden ist. An der festen Phase ist zudem ein zweiter Primärantikörper fixiert, welcher Human-Immunglobuline und bevorzugt menschliches sekretorisches IgA erkennt. Da *H.pylori*-Antigene in einer Stuhloder Speichelprobe mit dem körpereigenen menschlichen Immunsystem in Berührung gekommen sind, wurden einige oder alle *H. pylori*-Epitope bereits von menschlichen Immunglobulinen gebunden und sind für eine Bindung durch den ersten Primärantikörper gegen den Erreger nicht mehr zugänglich. Von den zweiten Primärantikörper gegen menschliches Immunglobulin werden aber dennoch die Erregerantigene in der Stuhl- oder Speichelprobe und Biopsiematerial, selbst wenn gegen diese bereits eine Immunreaktion besteht, an der festen Phase konzentriert und gebunden. Es werden also einerseits H.pylori-Antigene direkt gebunden und andererseits indirekt über ihre Bindung an sekretiertes menschliches Immunglobulin im Speichel oder Magendarmtrakt. Im zweiten Schritt wird dann gebundenes *H.pylori*-Antigen unmittelbar durch Anti-*H.pylori*-Sekundärantikörper detektiert. Über ein geeignetes System wird dann die Menge an gebundenem Sekundärantikörper quantifiziert.

Durch die hohe Spezifität des Senkundärantikörpers gegen *H.pylori*-Antigen werden falsch positive Befunde ausgeschlossen. Die Gesamtspezifität eines Doppelantikörperassays wird nämlich im Wesentlichen von der Spezifität des Sekundärantikörpers bestimmt. Dass im ersten Bindeschritt auch menschliche sekretorische Antikörper mit Erregerantigen gebunden werden, erhöht im Fall von *H. pylori* nicht nur die Empfindlichkeit, sondern auch die Bandbreite des Tests. Besonders erstaunlich ist, dass offenbar in einer erheblichen Zahl der Fälle alle immunogenen Epitope des *H. pylori* von der bestehenden menschlichen Immunreaktion erkannt werden. Offenbar ist bei Helicobacter-Organismen die Zahl der immunogenen Epitope begrenzt, so dass die vom menschlichen Immunsystem und von den tierischen Primär- und Sekundärantikörpern des Doppelantikörper-Bindungsassays erkannten Erregerepitope oftmals identisch sind. Dies gilt insbesondere bei einer *H.pylori*-Besiedelung der Mundhöhle und für die Spätphase einer Eradikationstherapie, in der die menschliche Immunreaktion die nunmehr verminderte Menge an *H.pylori*-Epitopen, die in der Stuhloder Speichelprobe noch vorhanden sind, abdeckt. Durch das erfindungsgemäße Testprinzip lassen sich geringe und auch maskierte Mengen *H. pylori*-Antigen erfassen.

Das Vorkommen von *Helicobacter pylori* im Speichel erregte bislang nur wissenschaftliches Interesse hinsichtlich des mutmaßlichen Übertragungsweges (Namavar F. et al., *Eur. J. Clin. Microbiol. Infect. Dis.,* 1995, 14(3), pp. 234-7; Shimada T. et al., *Lancet*, 1994, 343(8913), pp. 1636-7). Durch das erfindungsgemäße Verfahren wird die untere Nachweisgrenze für *H. pylori* nun so tief gelegt, dass auch eine Untersuchung des Speichels die Diagnose einer *H.pylori*-Infektion erlaubt. Insbesondere für die Therapiekontrolle ist es wichtig, den Speichel des Patienten auf *H. pylori*-Antigene zu untersuchen. Die Untersuchungen der Anmelderin ergaben, dass sich im Mundraum - vermutlich unter Metallplomben und Zahnprothesen, etc.- *H. pylori*-Infektionsnester befinden können, welche eine herkömmliche medikamentöse Eradikationstherapie überdauern können und nach Beendigung der Therapie zu einem erneuten Befall der Magenschleimhaut führen. Eine Eradikationstherapie kann somit nur als abgeschlossen gelten, wenn sowohl in der Stuhl- als auch in der Speichelprobe keine *H. pylori*-Antigene mehr nachgewiesen werden können.

Das erfindungsgemäße Sandwich-Testprinzip mit einem zweiten Primärantikörper gegen menschliches Immunglobulin, insbesondere gegen menschliches sekretorisches IgA, ist nicht auf die Verwendung auf einer Mikrotiterplatte beschränkt. Es kann auf vollautomatisierte Verfahren adaptiert werden, welche mit beschichteten Beads bzw. Partikeln arbeiten. Das erfindungsgemäße Prinzip mit einem zweiten Primärantikörper gegen sekretorisches Human-IgA eignet sich allgemein für die Diagnostik von Erregern, die im Mundraum oder im Magendarmtrakt siedeln und eine massive Immunreaktion hervorrufen.

Im Allgemeinen ist bevorzugt, wenn der erste Primärantikörper ein Pool polyklonaler Antikörper gegen verschiedene *H. pylori*-Stämme ist. Dieses gilt auch für den Sekundärantikörper, da dies die Sicherheit des Tests verbessert.

Die Erfindung und deren Vorteile werden nun an bevorzugten Ausführungsformen, Beispielen und mit Bezug auf die anliegenden Zeichnungen beschrieben. Es zeigt:

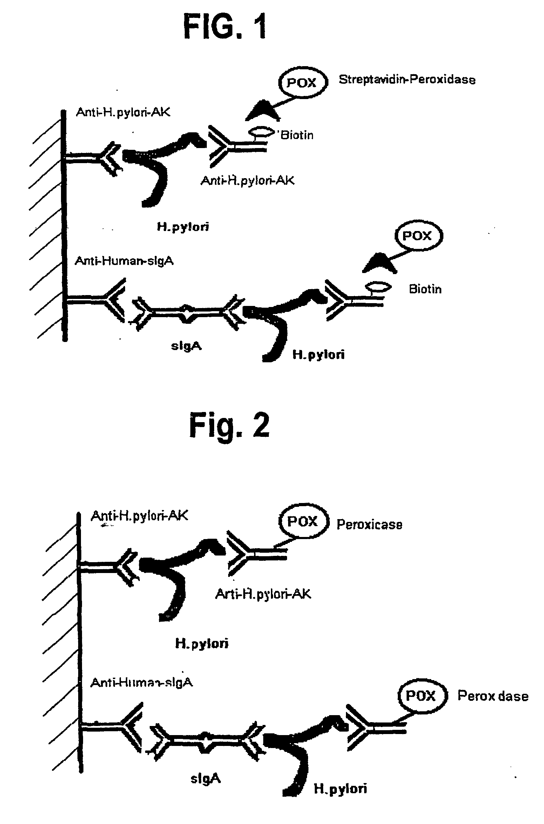

- Fig. 1: ein Prinzipschema des erfindungsgemäßen Immunoassays auf *H.pylori*-Antigen;
- Fig. 2: eine weitere Ausführungsform des erfindungsgemäßen Immunoassays;
- Fig. 3: graphische Darstellung der erfindungsgemäß bestimmten *H.pylori*-Antigen-Konzentration im Speichel vor und nach einer Eradikationsbehandlung.

### BEISPIEL 1 - Nachweis von H.pylori-Antigen in Stuhl und Speichel

*Vorbereitung der Stuhlprobe*: Es wurde ca. 100 mg Stuhl eingewogen und bei Raumtemperatur in 5 ml PBS-Waschpuffer mit 0,1% Triton X-100 (8 mM Na₂HPO₄, 15 mM M K₂H₂PO₄, 3 mM KCl, 12,5mM NaCl, 0,1% Triton X-100, 0,02% Thimerosal, pH 7,4) dispergiert. Die Dosierung und Homogenisierung der Stuhlproben erfolgte bevorzugt mit einem Probenvorbereitungssystem der Roche Diagnostik, Mannheim, DE (Best.-Nr. 745804). Das Homogenat wurde in einer Tischzentrifuge 10 Minuten bei 3000 Upm zentrifugiert, 1 ml Überstand in ein Eppendorf-Röhrchen überführt und 5 Minuten bei 13000 Upm in einer Eppendorf-Zentrifuge zentrifugiert. Der Überstand wurde direkt in den ELISA-Test eingesetzt.

*Vorbereitung der Speichelprobe:* Die Speichelprobe wurden 1:4 oder 1:5 je nach Viskosität in Assaypuffer verdünnt und unmittelbar in den Bindungsassay eingesetzt. Bei Speichelproben ist es vorteilhaft, wenn der Assaypuffer einen Protease-Inhibitor wie bspw. 5 mM PMSF enthält. Es können auch kommerzielle Proteaseinhibitoren wie Pefabloc SC (Roche Diagnostics) verwendet werden.

*Beschichtung der Mikrotiterplatte:* Die Vertiefungen einer Mikrotiterplatte wurden mit polyklonalen Antikörpern gegen *H.pylori*-Antigen und Human-Immunglobulin-A beschichtet. Hierzu wurde in jede Vertiefung jeweils 100 µg käuflicher polyklonaler Kaninchen-Anti-*H.pylori*-Antikörper (DAKO, Hamburg) gegeben, gelöst in 200 µl 60 mM NaCO₃, pH 9,6, und die Platte über Nacht bei 4°C inkubiert. Die Kaninchen-Anti-*H.pylori*-Antikörper-Lösung in den Vertiefungen wurde entfernt und jede Vertiefung mit 200 µl Waschpuffer (PBS, pH 7,4 mit 0,1% Triton X-100) gewaschen. Dann wurde in jede Vertiefung 100 µg Kaninchen-Anti-Human-sIgA-Antikörper (DAKO, Hamburg) gegeben, gelöst in 200 µl 60mM NaCO₃, pH 9,6 und die Platte eine Stunde bei Raumtemperatur inkubiert. Die Anti-Human-IgA-Antikörper-Lösung in den Vertiefungen wurden entfernt und jede Vertiefung erneut fünfmal mit je 250 µl Waschpuffer gewaschen.

Nach dem letzten Waschgang wurden die Vertiefungen der Mikrotiterplatte auf Saugpapier ausgeschlagen.

*Bindungsassay:* Die Bestimmungen erfolgten alle in Doppelwerten. 100 µl Standard und Probe wurde in Doppelwerten in die antikörperbeschichteten Vertiefungen der Mikrotiter pipettiert und eine Stunde unter Schütteln bei Raumtemperatur inkubiert. Die Lösungen wurden abgenommen und die Vertiefungen der Platte fünfmal mit je 250 µl Waschpuffer gewaschen. Nach dem letzten Waschgang wurde die Mikrotiterplatte auf Saugpapier trocken geschlagen.

*Bestimmung der Bindung:* Es wurde jeweils 100 µl Biotin-konjugierter polyklonaler Kaninchen-Anti-*H.pylori*-Antikörper (1:10000; von DAKO, Hamburg) oder Meerrettichperoxidase(HRP)-konjugierte polyklonaler Ziege-Anti-*H.pylori*-Antikörper (KPL, Kirkegaard and Perry Laboratories, Gaithersburg, MD; ein Gemisch aus polyklonalen Antikörper gegen die *H.pylori*-Stämme ATCC 43504, 43526, 43579) 1:1000 verdünnt in Waschpuffer, in die Vertiefungen gegeben und bei Raumtemperatur eine Stunde unter Rütteln inkubiert. Die Lösung wurde aus den Vertiefungen entfernt und jede Vertiefung fünfmal mit 200 µl Waschpuffer gewaschen.

*Quantitative Bestimmung:* Für die Farbreaktion wurde im Fall des Meerrettichperoxidase-konjugierten Ziege-Anti-*H.pylori*-Antikörpers 100 µl Tetramethylbenzidin(TMB)-Substratlösung (gebrauchsfertig von NOVUM Diagnostika GmbH, Dietzenbach, DE) in die Vertiefungen gegeben und nach etwa 20 Minuten die Farbentwicklung durch Zugabe von 50 µl 0,4M H₂SO₄ gestoppt. Im Fall des Biotin-gekoppelten Kaninchen-Anti-*H.pylori*-Antikörpers wurde 100 µl Meerrettichperoxidase-konjugiertes Streptavidin (DAKO), 1:10000 verdünnt in Waschpuffer, aufgetragen, 1 Stunde bei Raumtemperatur unter Schütteln inkubiert, fünfmal mit Waschpuffer gewaschen und erst dann das Chromogen hinzugefügt. Die Farbentwicklung wurde jeweils bestimmt durch Messung der Extinktion bei 450 nm. Die nachstehenden Tabellen 1 und 2 zeigen repräsentative Ergebnisse von Gesunden und *H.pylori*-infizierten Patienten, wobei die erfindungsgemäßen Bestimmungen an verschiedenen Tagen wiederholt wurden. Die Bestimmungen erfolgten wie angegeben mit unterschiedlichen Nachweissystemen bzw. Sekundärantikörpern.

### VERGLEICHSBEISPIEL 2

### Immunoassay ohne zweiten Antikörper gegen Human-Immunglobulin-A

Die Vorbereitungen der Stuhl- und Speichelproben und der Bindungsassay erfolgten exakt wie in Beispiel 1, nur dass die jeweiligen Vertiefungen der Mikrotiterplatte ausschließlich mit affinitätsgereinigtem, polyklonalen Kaninchen-Immunglobulin gegen *Helicobacter pylori* oder mit Kaninchen-Anti-Human-sIgA-Antikörper beschichtet wurde. Alle Wasch-, Beschichtungs- und Bindeschritte sowie die Farbentwicklungen erfolgten parallel auf der gleichen Mikrotiterplatte.

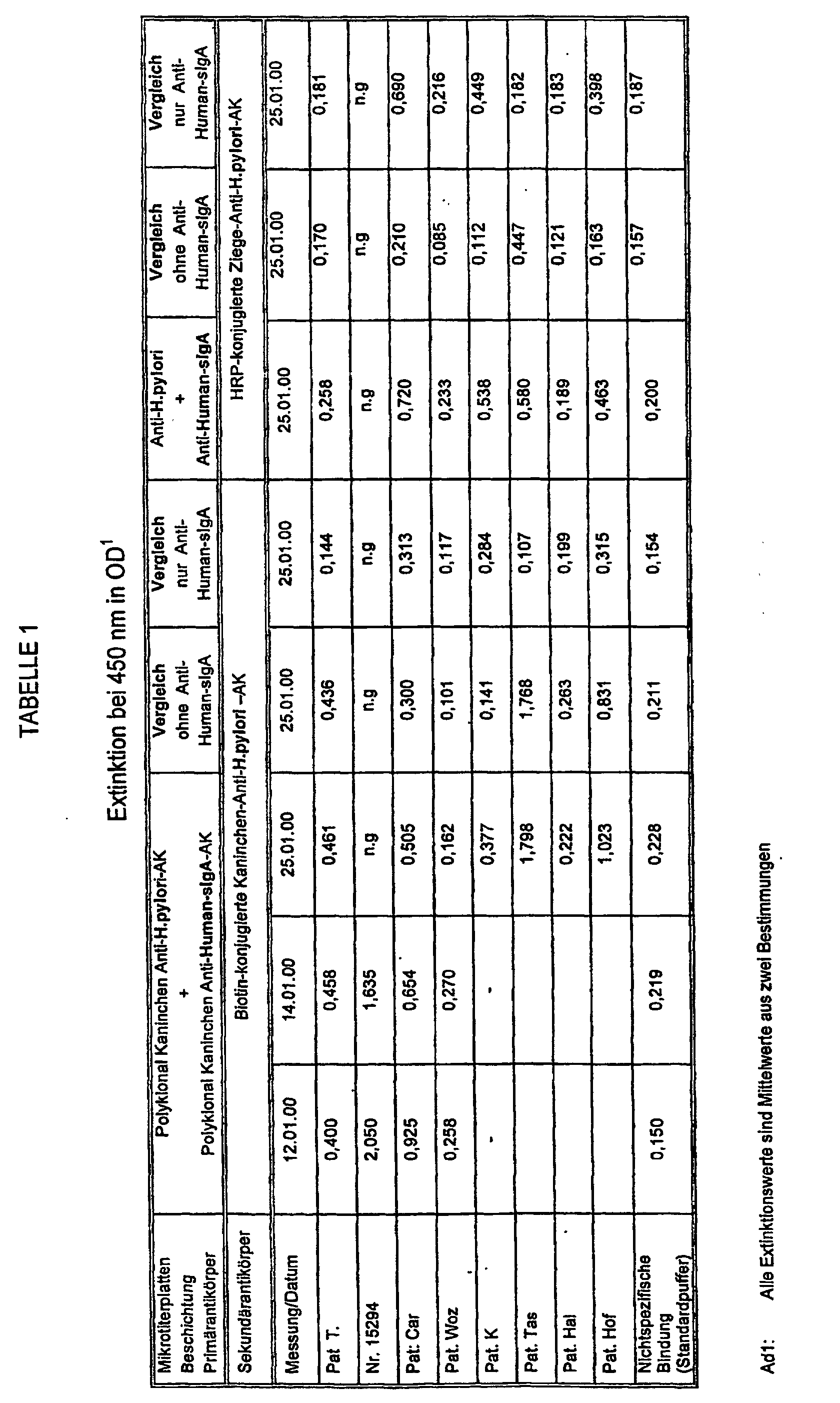

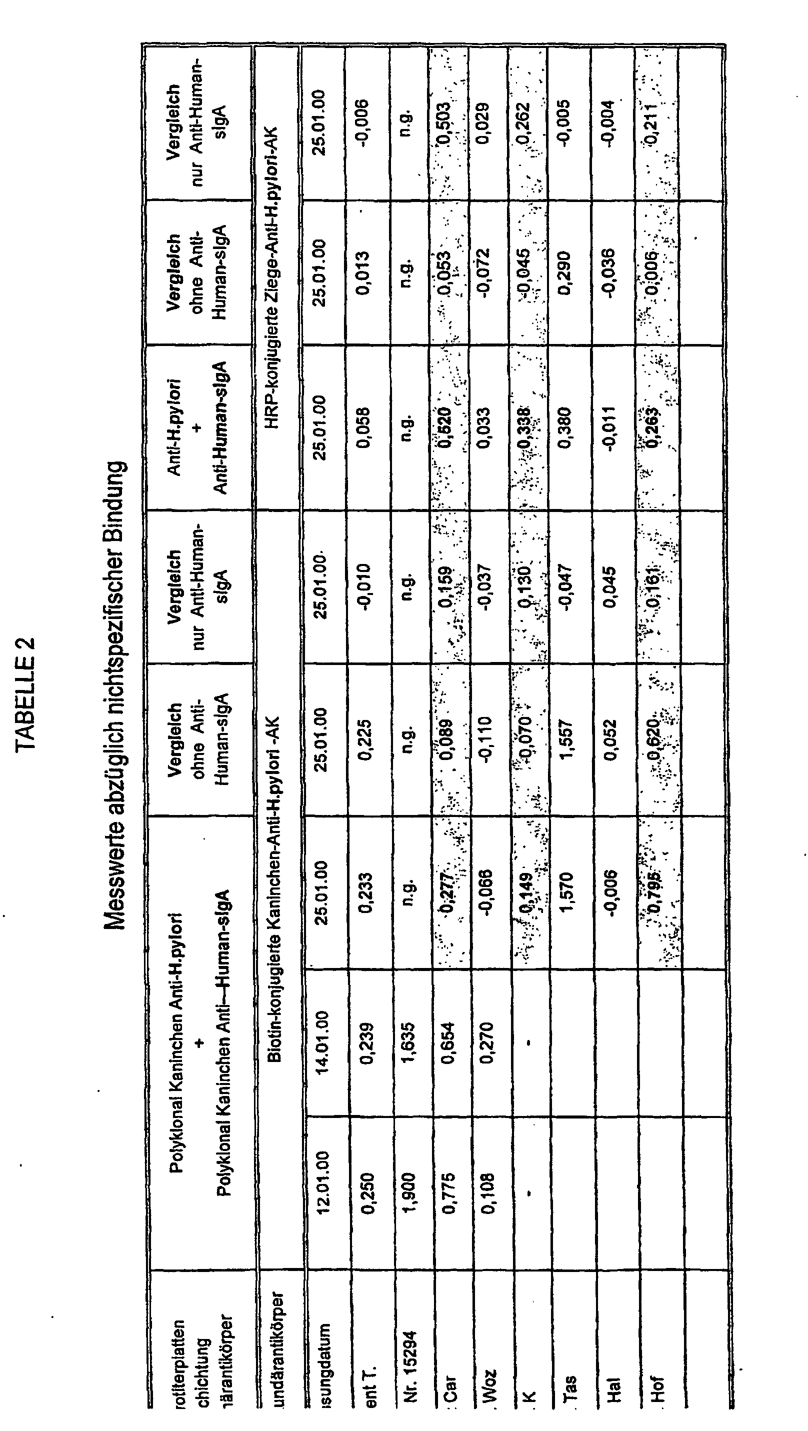

### DISKUSSION

Die Tabellen 1 und 2 zeigen, dass bei manchen Patienten die in der Stuhlprobe vorhandenen *H.pylori*-Antigene vollkommen vom körpereigenen Immunsystem maskiert waren und daher nicht vom analytischen Primärantikörper gegen den Erreger gebunden werden konnten. Bei einer solchen Maskierung der Antigene führt ein herkömmlicher Doppelantikörper-Immunoassay zu dem falschen Ergebnis, dass keine *H.pylori*-Infektion vorliegt bzw. nicht mehr vorliegt. Beim erfindungsgemäßen Verfahren wurden hingegen die vom menschliche Immunsystem bereits gebundenen bzw. maskierten *H.pylori*-Antigene vom zweiten Anti-Human-sIgA-Primärantikörpern an die feste Phase gebunden (siehe Tabelle 2, grau unterlegte Felder). Der Maskierungseffekt war nicht abhängig vom eingesetzten Sekundärantikörper und auch nicht davon, ob es sich bei der Probe um Speichel oder Stuhl handelte. Ferner legen die Ergebnisse nahe, dass sich die Primärbindungen von *H.pylori* an der festen Phase über Anti-HumansIgA-Antikörper bzw. Anti-H.pylori-Antikörper in vielen Fällen einander ausschließen, dass also deutlich eine Entweder-Oder-Situation hinsichtlich der Bindung gegeben war. Die mögliche Primärbindung der *H.pylori*-Antigene an die feste Phase über die Bindung an menschliches Immunglobulin-A ist somit essentieller Bestandteil der erfindungsgemäßen Diagnostik.

### Beispiel 3 - Speichelreihenuntersuchungen

Es wurde von 150 Patienten mit Verdacht auf eine H.pylori Infektion bzw. nach erfolgter Eradikationstherapie eine Speichelprobe entnommen und auf die Anwesenheit von H.pylori-Antigenen untersucht. Die Analytik erfolgte wie in Beispiel 1 angegeben mit der Ausnahme, dass der Assaypuffer zudem einen Proteaseinhibitor enthielt. Die Nachweisgrenze für Helicobacter-Antigen in Speichel liegt in dem vorstehend beschriebenen Testsystem (mit HRP-konjugiertem Ziege-Anti-*H.pylori*-Antikörper und Tetramethylbenzidin) bei etwa 2 pg H.pylori-Antigen pro Milliliter Assaypuffer. Durch Optimierung des gewählten zweiten Antikörpers gegen das Erregerantigen und Wahl eines Lumineszenz-Detektionssystems sollten Nachweisgrenzen 0,2 pg Antigen pro Milliliter und darunter erreicht sein.

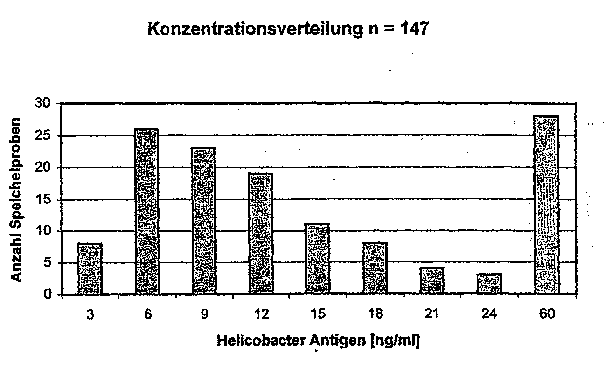

Das Ergebnis der Reihenuntersuchung wird in Figur 3 in einem Balkendiagramm zusammengefasst. Das Balkendiagramm zeigt, dass Patienten mit einer bestehenden H.pylori-Infektion im Speichel in der Regel mehr als 60 ng/ml *H.pylori*-Antigene besitzen. Die Untersuchung des Speichels ist daher geeignet für den Nachweis einer bestehenden H.pylori-Infektion. H.pylori-Antigenkonzentrationen unter 25 ng pro ml Speichel deuten hingegen auf eine unspezifische Kreuzreaktion mit anderen Erregern hin.

Damit wurde zum ersten Mal der Beweis erbracht, dass eine *H.pylori*-Infektion über eine vergleichsweise einfach durchzuführende immunologische Speicheluntersuchung nachgewiesen werden kann. Auch lässt sich so der Erfolg einer Eradikationstherapie vergleichsweise einfach verfolgen. Die bisherige Analytik des Speichels auf H.pylori hingegen erlaubte keine Diagnostik einer H.pylori-Infektion des Magen-Darmtrakts, sondern beruhten auf einer wissenschaftliche Untersuchungen des möglichen Übertragungsweges.

## Patentansprüche

1. Verfahren zum Nachweis von krankheitserregenden Organismen in Stuhl-, Speichel- und Sekretproben durch einen Doppelantikörper-Sandwich-Bindungsassay, **gekennzeichnet durch**
Aufnehmen oder Dispergieren der Probe mit Erregerantigen in einer Pufferlösung und Zusammenbringen der Pufferlösung mit einer festen Phase, an der mindestens zwei primäre Antikörper gebunden sind, von denen einer Erregerantigen und der andere menschliches Immunglobulin-A spezifisch bindet;
Waschen der festen Phase von nicht spezifisch gebundenen Proteinen und Zusammenbringen der festen Phase mit einem sekundären Antikörper, der spezifisch an Erregerantigen bindet, und Bestimmen der Menge an spezifisch gebundenem Sekundärantikörper.

2. Verfahren nach Anspruch 1, wobei der zweite Primärantikörper sekretorisches Human-IgA bindet.

3. Verfahren nach Anspruch 2, wobei der zweite Primärantikörper sekretorisches Human-IgA2 bindet.

4. Verfahren nach einem der Ansprüche 1 bis 3, wobei der Sekundärantikörper konjugiert ist mit einer Gruppe, ausgewählt aus Biotin, Fluroescein, Rhutenium, Europium, Goldpartikel, alkalischer Phosphatase, Galactosidase und Meerrettichperoxidase .

5. Verfahren nach einem der Ansprüche 1 bis 4, wobei der erste Primärantikörper und der Sekundärantikörper *Helicobacter pylori*-Antigene bindet.

6. Verfahren nach einem der Ansprüche 1 bis 5, wobei der erste Primärantikörper eine Mischung polyklonaler und/oder monoklonaler Antikörper gegen verschiedene *Helicobacter pylori*-Stämme ist.

7. Verfahren nach einem der Ansprüche 1 bis 6, wobei der Sekundärantikörper eine Mischung polyklonaler und/oder monoklonaler Antikörper gegen verschiedene *Helicobacter pylori*-Stämme ist.

8. Verfahren nach einem der Ansprüche 1 bis 4, **dadurch gekennzeichnet, dass** der Erreger *Campylobacter* ist.

9. Verfahren nach einem der Ansprüche 1 bis 4, **dadurch gekennzeichnet, dass** der Erreger *Helicobacter heilmanii* ist.

10. Verfahren nach einem der Ansprüche 1 bis 4, **dadurch gekennzeichnet, dass** der Erreger eine massive Immunreaktion im menschlichen Körper hervorruft.

11. Verfahren nach einem der vorhergehenden Ansprüche 1 bis 10 zur Diagnose von verbliebenen Erregern im Mundraum nach einer medikamentösen Eradikationstherapie.

## Claims

1. Method of detecting pathogenic organisms in faecal, salivary and secretory samples by means of a double-antibody sandwich-binding assay, **characterized by**
taking up or dispersing a sample having pathogen antigen in a buffer solution and bringing together the buffer solution with a solid phase to which at least two primary antibodies are bound, of which one specifically binds a pathogen antigen and the other specifically binds human immunoglobulin-A;
washing the solid phase of not specifically bound proteins and bringing together the solid phase with a secondary antibody which specifically binds to pathogen antigen, and determining the quantity of specifically bound secondary antibody.

2. Method according to claim 1, wherein the second primary antibody binds secretory human-IgA.

3. Method according to claim 2, wherein the second primary antibody binds secretory human-IgA2.

4. Method according to any of claims 1 to 3, wherein the secondary antibody is conjugated with a group selected from biotin, fluorescene, ruthenium, europium, gold particles, alkaline phosphatase, galactosidase, and horseradish peroxidase.

5. Method according to any of claims 1 to 4, wherein the first primary antibody and the secondary antibody bind *Helicobacter pylori* antigens.

6. Method according to any of claims 1 to 5, wherein the first primary antibody is a mixture of polyclonal and/or monoclonal antibodies against various strains of *Helicobacter pylori*.

7. Method according to any of claims 1 to 6, wherein the secondary antibody is a mixture of polyclonal and/or monoclonal antibodies against various strains of *Helicobacter pylori*.

8. Method according to any of claims 1 to 4, **characterized in that** the pathogen is *Campylobacter*.

9. Method according to any of claims 1 to 4, **characterized in that** the pathogen is *Helicobacter heilmanii*.

10. Method according to any of claims 1 to 4, **characterized in that** the pathogen brings about a massive immune reaction in the human body.

11. Method according to any of preceding claims 1 to 10 for the diagnosis of remaining pathogens in the mouth after a medical eradication treatment.

## Revendications

1. Procédé de détection d'organismes générant des maladies dans les échantillons de selles, de salive et d'excréments par un essaie de liaison sandwich à double anti-corps, **caractérisé par**
la réception ou la dispersion des échantillons avec antigènes de pathogènes dans une solution de tampon et la mise en commun de la solution de tampon avec une phase solide à laquelle sont reliés au moins deux anti-corps primaires, parmi lesquels l'un lie l'antigène de pathogène et l'autre lie l'immunoglobine A humain de façon spécifique;
lavage de la phase solide des protéines qui ne sont pas spécifiquement liées et mises en commun de la phase solide avec un anti-corps secondaire qui lie de façon spécifique à des antigènes de pathogènes et détermination de la quantité d'anticorps secondaires liés de façon spécifique.

2. Procédé selon la revendication 1 où le deuxième anticorps primaire lie des sécrétions humaines IgA.

3. Procédé selon la revendication 2, où le deuxième anticorps primaire lie des sécrétions humaines IgA2.

4. Procédé selon une des revendications 1 à 3, où l'anticorps secondaire est conjugué à un groupe choisi parmi de la biotine , de la fluorescéine, du ruthénium, de l'europium, des particules d'or, du phosphatase alcalin, du galactosidase et du peroxydase de raifort.

5. Procédé selon une des revendications 1 à 4, où le premier anticorps primaire et le deuxième anticorps lie des antigènes de *helicobacters pylori*.

6. Procédé selon une des revendications 1 à 5, où le premier anticorps primaire est un mélange d'anticorps polyclonals et/ou des anticorps monoclonals contre différentes souches *d'helicobacters pylori*.

7. Procédé selon une des revendications 1 à 6, où l'anticorps secondaire est un mélange d'anticorps polyclonals et/ou monoclonals contre différentes souches *d'helicobacters pylori*.

8. Procédé selon une des revendications 1 à 4, **caractérisé en ce que** le pathogène est un *campylobacter*.

9. Procédé selon une des revendications 1 à 4, **caractérisé en ce que** le pathogène est un *helicobacter heilmanii*.

10. Procédé selon une des revendications 1 à 6, **caractérisé en ce que** le pathogène provoque une réaction d'immunité massive dans le corps humain.

11. Procédé selon une des revendications précédentes 1 à 10 pour diagnostiquer des pathogènes restant dans la bouche après une thérapie d'éradication médicamenteuse.
